(19) Europäisches Patentamt
European Patent Office
Office européen des brevets

(11) Veröffentlichungsnummer : **0 140 120 B1**

(12) **EUROPÄISCHE PATENTSCHRIFT**

(45) Veröffentlichungstag der Patentschrift : **11.11.87**

(51) Int. Cl.⁴ : **C 07 D209/48**, C 08 L 69/00

(21) Anmeldenummer : **84111049.7**

(22) Anmeldetag : **17.09.84**

(54) **Neue Flammschutzmittel, ihre Hersteelung und ihre Verwendung zur Flammfestausrüstung von Polycarbonaten.**

(30) Priorität : **26.09.83 DE 3334822**

(43) Veröffentlichungstag der Anmeldung : **08.05.85 Patentblatt 85/19**

(45) Bekanntmachung des Hinweises auf die Patenterteilung : **11.11.87 Patentblatt 87/46**

(84) Benannte Vertragsstaaten : **DE FR GB IT NL**

(56) Entgegenhaltungen :
**EP-A- 0 086 353**
**EP-A- 0 103 922**
**DE-A- 2 937 877**
**DE-A- 3 346 814**
**GB-A- 1 400 728**

(73) Patentinhaber : **BAYER AG**
**Konzernverwaltung RP Patentabteilung**
**D-5090 Leverkusen 1 Bayerwerk (DE)**

(72) Erfinder : **Kress, Hans-Jürgen, Dr.**
**Scheiblerstrasse 111**
**D-4150 Krefeld (DE)**
Erfinder : **Kircher, Klaus, Dr.**
**Alfred-Kubin-Strasse 3**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 140 120 B1

Jouve, 18, rue St-Denis, 75001 Paris, France

**Beschreibung**

Gegenstand der vorliegenden Erfindung sind Phthalimidverbindungen der Formel (I)

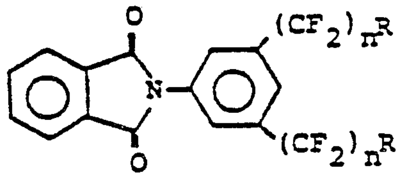

(I)

worin

n eine ganze Zahl von 1-12, vorzugsweise von 1 bis 4 und insbesondere von 1 bis 2, und

R = H oder F

sind, wobei für n = 1 R nicht F ist.

Gegenstand der vorliegenden Erfindung ist außerdem die Herstellung von Phthalimiden der Formel (I), die dadurch gekennzeichnet ist, daß Phthalsäureanhydrid mit einem Amin der Formel (II)

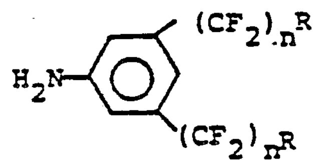

(II)

worin

n eine ganze Zahl von 1-12, vorzugsweise von 1 bis 4 und insbesondere von 1 bis 2, und

R = H oder F sind, wobei für n = 1 R nicht F ist,

in äquimolaren Mengen bei etwa 117 °C und unter Mitverwendung von Eisessig als Lösungsmittel und Cyclohexan als Wasserschlepper umgesetzt wird.

Die Amine der Formel (II) sind literaturbekannt oder nach literaturbekannten Verfahren erhältlich, wie sie beispielhaft für das 3,5-Bis-tetrafluorethylaninin im US-Patent 2 876 251 beschrieben sind.

Die Menge an einzusetzendem Lösungsmittel beträgt auf 1 Mol des erfindungsgemäßen Anilins, bezogen ca. 2,5 l Eisessig ; die entsprechende Menge an Wasserschlepper beträgt ca. 300 ml.

Die neuen Phthalimidverbindungen sind in Kombination mit den bekannten Flammschutzmitteln für Polycarbonate, den Alkalisalzen organischer oder anorganischer Säuren, geeignete Synergisten zur Verbesserung der Flammwidrigkeit von thermoplastischen, verzweigten, aromatischen Polycarbonaten, die nur aus halogenfreien phenolischen Komponenten hergestellt sind.

Gegenstand der vorliegenden Erfindung sind somit auch Flammschutzmittelkombinationen bestehend aus

a) 0,1 bis 1 Gewichtsteil eines Phthalimids der Formel (Ia)

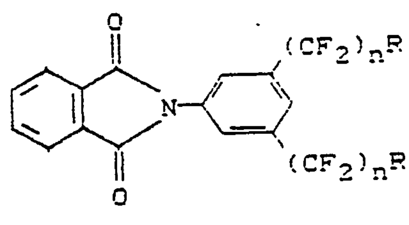

(Ia)

worin

n eine ganze Zahl von 1-12 und

R = H oder F sind

b) 0,02 bis 2 Gewichtsteilen eines Alkalisalzes einer organischen oder anorganischen Säure, insbesondere eines Natrium-, Kalium- oder Lithiumsalzes.

Als Flammschutzmittel geeignete Alkalisalze von organischen oder anorganischen Säuren sind beispielsweise in den deutschen Offenlegungsschriften Nr. 2 703 710 Nr. 2 918 882 und Nr. 2 918 883 genannt.

Gegenstand der vorliegenden Erfindung ist außerdem die Verwendung der erfindungsgemäßen Flammschutzmittelkombination zu Flammfestausrüstung von thermoplastischen, verzweigten, aromatischen Polycarbonaten aus halgenfreien phenolischen Komponenten in Mengen von 0,1 bis 1 Gew.-%, bezogen auf thermoplastisches, verzweigtes, aromatisches Polycarbonat, an Phthalimid der Formel (Ia) und 0,02 bis 2 Gew.-%, bezogen auf thermoplastisches, verzweigtes, aromatisches Polycarbonat, an

Alkalisalz einer organischen oder anorganischen Säure.

Gegenstand der vorliegenden Erfindung ist außerdem ein Verfahren zur Flammfestausrüstung von thermoplastischen, verzweigten, aromatischen Polycarbonaten aus halogenfreien phenolischen Komponenten, das dadurch gekennzeichnet ist, daß die Einarbeitung der erfindungsgemäßen Flammschutzmittelkombination als Einzelkomponenten oder als Gemisch durch Mischen und anschließende Granulierung über einen Doppelwellenextruder bei einer Massetemperatur von 280-310 °C erfolgt.

Die optimalen Verarbeitungsbedingungen sind derart, daß bei einer Umdrehungszahl von 80-100 Umdrehungen/min ein Durchsatz von 5 kg/h erzielt wird.

Das verwendete Aggregat war ein Extruder der Firma Werner und Pfleiderer mit der Bezeichnung ZSK 32.

Gegenstand der vorliegenden Erfindung sind außerdem thermoplastische Formmassen auf Basis aromatischer, verzweigter, thermoplastischer Polycarbonate aus halogenfreien phenolischen Komponenten mit einem Gehalt von 0,1 bis 1 Gew.-% an Phthalimid der Formel (Ia) und von 0,02 bis 2 Gew.-% an Alkalisalz einer anorganischen oder organischen Säure, wobei sich die beiden Bereiche der Gewichtsprozente jeweils auf thermoplastisches, verzweigtes, aromatisches Polycarbonat ohne sonstige Zusätze beziehen.

Unter aromatischen, verzweigten, thermoplastischen Polycarbonaten aus halogenfreien phenolischen Komponenten ist zu verstehen, daß die für die Herstellung der Polycarbonate einzusetzenden Diphenole, Monophenole und Trisphenole, Tetraphenole oder sonstige Verzweiger keine Halogensubstituenten haben. Selbsverständlich können diese Polycarbonate, sofern sie beispielsweise nach dem Phasengrenzflächenverfahren unter Verwendung von Phosgen hergestellt sind, noch geringe Rest-ppm-Mengen an unverseiftem Chlor enthalten. Bei der nachfolgenden Charakterisierung der Polycarbonate als « halogenfrei » sollen Reste an derartigem verseifbaren Halogen unberücksichtigt bleiben.

Mit Hilfe dieser neuen Flammschutzmittel sind somit chlor- und bromfreie flammgeschützte Polycarbonate mit einer UL 94 V-Klassifizierung möglich. Zusätzlich sind die erfindungsgemäßen Phthalimide der Formel (I) durch niedrige Flüchtigkeit bei normalen Polycarbonatverarbeitungsbedingungen gekennzeichnet.

Die erfindungsgemäßen Polycarbonatformassen erreichen gemäß Underwriters' Laboratories Inc., Bulletin 94, Verbrennungstests zur Klassifizierung von Materialien (nachfolgend UL 94 bezeichnet), bei Prüfkörpern mit 127 × 12,7 × 3,2 mm (1/8")- bzw. 127 × 12,7 × 1,6 mm (1/16")-Maßen eine Einstufung in die Brandklasse VO, d. h. sie sind nicht abtropfend und besitzen eine durchschnittliche Nachbrennzeit von ≤ 5 s.

Prüftkörper mit 127 × 12,7 × 0,8 mm (1/32")-Maßen erreichen eine Einstufung in die Brandklasse V 1, das heißt, sie sind nicht abtropfend und besitzen eine durchschnittliche Nachbrennzeit von ≤ 25 s.

Bekannt ist, die Flammfestigkeit von Polycarbonaten durch Alkalisalzzusätze zu verbessern, wobei die Polycarbonate sowohl halogenfrei als auch halogensubstituiert sein können.

(Siehe beispielsweise DT-OS 1 930 257, DT-OS 2 049 358, DT-OS 2 112 987, DT-OS 2 1249 311, DT-OS 2 253 6072, DE-OS 2 458 968, 2 461 063, 2 461 146 und 2 461 077).

Bekannt ist auch, die Flammfestigkeit von Polycarbonaten durch Gemische von organischen Chlorverbindungen und gewissen anorganischen Salzen zu verbessern (s. beispielsweise DE-OS 2 013 496 ; als organische Chlorverbindungen wird u. a. Tetrachlorphthalsäureanhydrid als geeignet genannt).

Bekannt ist auch, mit bromierten Phthalimiden Polycarbonate flammwidrig zu machen (siehe US-Patent 3 873 587).

Bekannt ist auch der Einsatz von Phthalimidverbindungen in Kombination mit Alkalisalzzusätzen, wobei ausschlielich halogenierte Phthalimide eingesetzte werden (DOS 2 707 928, 2 740 850, 2 703 710).

Bekannt ist auch, organische Halogenverbindungen wie halogenierte Phthalimide in Kombination mit Alkalisalzen und Stoffen, die die Abtropfneigung von Polycarbonaten reduzieren, für die Flammfestausrüstung von Polymerlegierungen auf Basis von Polycarbonaten einzusetzen. (Siehe DE-OS 2 918 882 und DE-OS 2 918 883).

Bekannt ist auch, Formmassen aus verzweigten aromatischen Polycarbonaten zusammen mit Alkalisalzen und halogenierten Phthalimiden und einem zusätzlichen Bromgehalt für extreme Beflammungsbedingungen flammwidrig auszurüsten (siehe De-OS 3 203 905).

In den genannten Literaturstellen werden jedoch unseres Erachtens die Phthalimide der Formel (Ia) weder vorweggenommen noch nahegelegt, noch deren Verwendung als Flammschutzsynergist für halogenfreie Polycarbonate.

Es ist auch bislang keine Additivkombination bekannt, die nach Zumischen von so geringen Gewichtsmengen eines chlor- und bromfreien Phthalimids bereits Polycarbonatformassen der Brandklassen V 0 gemäß UL 94 bei 1/8" sowie 1/16" und V 1 bei 1/32" Wandstärke ergeben.

Die erfindungsgemäß beanspruchte Zumischung von Phthalimiden der allgemeinen Formel (Ia) ist deshalb besonders vorteilhaft, weil Verbindungen dieser Substanzklassen sehr thermostabil, schwerflüchtig, verseifungsstabil und in Polycarbonat gut einmischbar sind.

Geeignete Alkalisalze anorganische Säuren im Sinne der Erfindung sind beispielsweise solche von anorganischen Protonsäuren. Anorganische Protonsäuren im Sinne der Erfindung sind Brönsted-Säuren, die Alkalisalze bilden können (zum Ausdruck « Brönsted-Säure » vgl. Fieser & Fieser « Organic

Chemistry », 1965, S. 595, Interscience Publishers N.Y., USA), wie zum Beispiel meta-, ortho- oder pyro-Phosphorsäure und Protonensäuren komplexer Fluormetallverbindungen.

Geeignete Alkalisalze organischer Säuren sind im Sinne der Erfindung solche von organischen Brönsted-Säuren mit mindestens einem Kohlenstoffatom, die Alkalisalze bilden können. Solche gegebenenfalls substituierten organischen Säuren können OH- oder NH-acide Verbindungen sein, wie beispielsweise Sulfonsäuren, Phosphonsäuren, Thiophosphonsäuren, NH-acide Sulfonamide oder Sulfonimide. Sie müssen mindestens ein C-Atom haben und können vorzugsweise zwischen 2 und 30 C-Atome enthalten.

Die erfindungsgemäß geeigneten Alkalisalze sollen vorzugsweise einen pH-Wert zwischen 5 und 9, insbesondere zwischen 6,5 und 7,5 haben, gemessen an 1 gew.-%igen Lösungen oder Suspensionen der Salze in Wasser bei 20 °C.

Bevorzugte Alkalisalze sind die Kalium-, Natrium und Lithiumsalze, insbesondere die Kaliumsalze.

Bevorzugte Alkalisalze organischer Säuren sind die Natrium-, Kalium- und Lithiumsalze, insbesondere jedoch die Kaliumsalze von organischen Sulfonsäuren und Phosphonsäuren, deren organische Reste gegebenenfalls durch Halogene, wie Fluor, Chlor oder Brom, substituiert sein können. Beispielsweise seien genannt : Natrium- oder Kalium-perfluorbutansulfat, Natrium- oder Kalium-perfluormethansulfonat, Natrium- oder Kalium-2,5-dichlorbenzolsulfonat, Natrium- oder Kalium-2,4,5-trichlorbenzolsulfonat, Natrium- oder Kalium-(4-chlorphenyl)-phosphonat, Natrium- oder Kalium-methylphosphonat, Natrium- oder Kalium-(2-phenylethylen)-phosphonat und Lithium-phenylphosphonat.

Bevorzugte Alkalisalze anorganischer Säuren sind die Natrium-, Kalium- und Lithiumsalze, insbesondere jedoch die Kalium-Salze von Protonensäure-Komplexe-Fluormetallverbindungen sowie von meta-, ortho oder pyro-Phosphorsäure.

Beispielsweise seien genannt : Trinatrium- oder Trikalium-hexafluoroaluminat, Dinatrium- oder Dikalium-hexafluorotitanat, Dinatrium- oder Dikalium-hexafluorosilikat, Dinatrium- oder Dikalium-hexafluorozirkonat, Natrium- oder Kalium-pyrophosphat, Natrium- oder Kalium-metaphosphat, Natrium- oder Kalium-tetrafluoroborat, Natrium- oder Kalium-hexafluorophosphat und Natrium- oder Lithiumphosphat.

Besonders geeignete Salze sind : Kalium- oder Natrium-perfluorbutansulfonat, Kalium- oder Natrium-2,5-dichlorbenzolsulfonat, Kalium- oder Natrium-2,4,5-trichlorbenzolsulfonat, Kalium-hexafluoroaluminat, Kalium-pyrophosphat, Kalium-methylphosphonat, Natrium-hexafluoroaluminat und Lithiumphenylphosphonat.

Ebenfalls sind Mischungen der Salze untereinander geeignet.

Halogenfreie aromatische, verzweigte, thermoplastische Polycarbonate im Sinne der vorliegenden Erfindung sind durch Umsetzung von halogenfreien Diphenolen, insbesondere von Dihydroxydiarylalkanen, mit Phosgen oder Diestern der Kohlensäure erhältliche Polykondensate, wobei außer den unsubstituierten Dihydroxydiarylalkanen auch solche geeignet sind, deren Arylreste in o- und/oder m-Stellung zur Hydroxylgruppe Alkylgruppen tragen, und die durch den Einbau von Mengen zwischen 0,05 und 2,0 Mol-% (bezogen auf eingesetzte Diphenole) an drei- oder mehr als dreifunktionellen Verbindungen, beispielsweise solchen mit drei oder mehr als drei phenolischen Hydroxygruppen verzweigt sind.

Polycarbonate dieser Art und deren Herstellung sind z. B. in den deutschen Offenlegungsschriften 1 570 533, 1 595 762, 2 116 974, 2 113 347, der britischen Patentschrift 1 079 821, der US-Patentschrift 3 544 514 und in der deutschen Offenlegungsschrift 2 500 092 beschrieben.

Die halogenfreien aromatischen, verzweigten, thermoplastischen Polycarbonate haben mittlere Gewichtsmittel-Molekulargewichte Mw zwischen 15 000 und 100 000, vorzugsweise zwischen 20 000 und 80 000, ermittelt durch Messung der rel. Viskosität in $CH_2Cl_2$ bei 25 °C und einer Konzentration von 0,5 g/100 ml nach entsprechender Eichung.

Geeignete halogenfreie Diphenole sind z. B. Hydrochinon, Resorcin, 4,4'-Dihydroxydiphenyl, Bis-(hydroxy-phenyl)-alkane wie beispielsweise $C_1$-$C_8$-Alkylen- bzw. $C_2$-$C_8$-Alkylidenbisphenole, Bis-(hydroxyphenyl)-cycloalkane wie beispielsweise $C_5$-$C_{15}$-Cycloalkylen- bzw. $C_5$-$C_{15}$-Cycloalkylidenbisphenole, Bis-(hydroxy-phenyl)-sulfide, -ether, -ketone, -sulfoxide oder -sulfone. Ferner $\alpha,\alpha'$-Bis-(hydroxyphenyl)-diisopropylbenzol sowie die entsprechenden kernalkylierten Verbindungen. Bevorzugt sind Polycarbonate auf Basis Bis-(4-hydroxy-phenyl)-propan-2,2 (Bisphenol A), Bis-(4-hydroxy-3,5-dimethylphenyl)-propan-2,2 (Tetramethylbisphenol A), Bis-(4-hydroxy-phenyl)-cyclohexan-1.1 (Bisphenol Z) sowie auf Basis von Dreikernbisphenolen wie $\alpha,\alpha'$-Bis-(4-hydroxyphenyl)-p-diisopropylbenzol.

Weitere für die Herstellung der Polycarbonate geeignete halogenfreie Diphenole sind in den US-Patenten 3 028 365 und 3 275 601 beschrieben.

Einige der verwendbaren Verbindungen mit drei oder mehr als drei phenolischen Hydroxygruppen sind beispielsweise Phloroglucin, 4,6-Dimethyl-2,4,6-tri-(4-hydroxyphenyl)-heptan-2,4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-[4,4-bis-(4-hydroxyphenyl)-cyclohexyl]-propan, 2,4-Bis-(4-hydroxyphenyl-isopropyl)-phenol, 2,6-Bis-(2'-hydroxy-5'-methyl-benzyl)-4-methylphenol, 2-(4-Hydroxyphenyl)-2-(2,4-dihydroxyphenyl)-propan, Hexy-(4-(4-hydroxyphenyl-isopropyl)-phenyl)-ortho-terephthalsäureester, Tetra-(4-hydroxyphenyl)-methan, Tetra-(4-(4-hydroxyphenylisopropyl)-phenoxy)-methan und 1,4-Bis-((4',4''-dihydroxytriphenyl)-methyl)-benzol. Einige der sonstigen dreifunktionellen Verbindungen sind 2,4-Dihydroxybenzoesäure, Trimesinsäure, Cyanurchlorid, 3,3-Bis-(4-hydroxyphenyl)-2-oxo-2,3-dihydroindol und 3,3-Bis-(4-hydroxy-3-methyl-phenyl)-2-oxo-2,3-dihydroindol.

Geeignete Kettenabbrecher zur Regulierung des Molekulargewichts sind beispielsweise in bekannter Weise Phenol und Alkylphenole, die in den bekannten Mengen eingesetzt werden.

Die Herstellung der aromatischen, verzweigten, thermoplastischen Polycarbonate erfolgt in bekannter Weise beispielsweise nach dem Phasengrenzflächenverfahren oder nach dem Verfahren in homogener Lösung. Auch nach dem bekannten Umesterungsverfahren können die aromatischen, thermoplastischen Polycarbonate hergestellt sein.

Besonders bevorzugte Polycarbonate im Sinne der vorliegenden Erfindung sind verzweigte Polycarbonate auf Basis Bisphenol-A mit einem Verzweigergehalt von 0,3 bis 1,0 Mol-%, bezogen auf Mole Bisphenol-A.

Geeignete Amine zur Herstellung der Penthalimide der Formel (Ia) sind z. B.

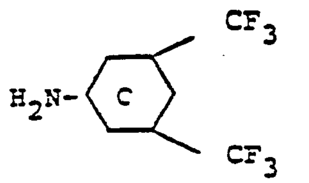

bzw.

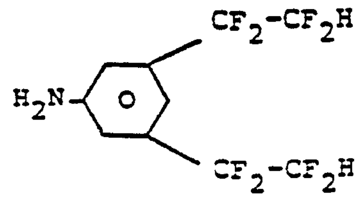

Sie sind beschrieben in Maginnity et al., J. Am. Soc. 73 (1951) S. 3579 bzw. in US-PS 2 876 251.

Geeignete Phthalimidverbindungen der Formel (Ia) sind z. B.

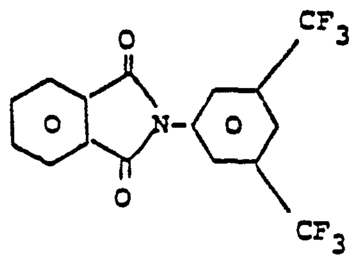

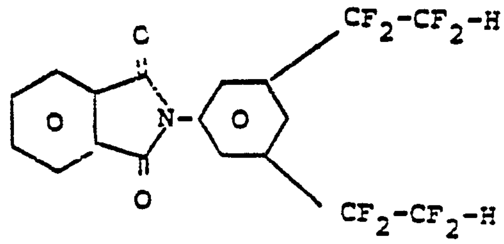

Sie sind nach dem Verfahren des Beispiels sowie nach den eingangs erwähnten allgemeinen Verfahrensbedingungen herstellbar.

Die erfindungsgemäßen Flammschutzmittelkombinationen können durch Mischen der Einzelkomponenten bzw. als Konzentrat in Polycarbonat vorab hergestellt werden und beispielsweise bis zur Verwendung gelagert werden.

Die Einarbeitung der neuen Flammschutzmittelkombination kann einzeln in Form ihrer Komponenten oder gemeinsam in die Polycarbonate erfolgen, beispielsweise durch Mischung und anschließende Granulierung des Materials über einen Doppelwellenextruder bei 280 bis 310 °C.

Die erfindungsgemäßen Formmassen auf Basis Polycarbonat und Flammschutzmittelkombination können noch andere, in der Polycarbonatchemie übliche Additive enthalten, wie beispielsweise Pigmente, Farbstoffe, Füllstoffe, Stabilisatoren oder Entformungsmittel.

Die erfindungsgemäßen Formmassen können zu Formteilen oder Folien verarbeitet werden.

Die Herstellung von Formkörpern erfolgt nach dem Spritzgießverfahren bei einer Temperatur von 300-310 °C.

Die erfindungsgemäßen Formmassen können eingesetzt werden z. B. im Elektrobereich für Schalterblenden, Steckdosen, Steckerleisten, Schaltkästen usw., im Haushaltssektor für Gehäuseteile von Bügeleisen, Kaffeemaschinen und im Großgerätebereich z. B. für Computergehäuseteile.

Wegen der älteren nationalen Schutzerechte gemäß DE-A 33 46 814 und der entsprechenden Äquivalente in England Nr. 213 3006, in Frankreich Nr. 25 38 386 und in den Niederlanden 83/44 06, wurden in den Patentansprüchen 1 und 2 ein Disclaimer aufgenommen.

Beschreibung des Verbrennungstests

Nach dem UL 94-Test (Underwriters Laboratories, Inc.) werden Polycarbonatproben zu Stäben der Abmessungen 127 × 12,7 × 0,8 (oder 1,6 oder 3,2) mm (5,00 × 0,05 × 1/32) (or 1/16 or 1/8 inches) geformt. Die Stäbe werden vertikal so montiert, daß die Unterseite des Probekörpers sich 305 mm über einem Streifen Verbandstoff befindet. Jeder Probestab wird einzeln mittels zweier aufeinanderfolgender Zündvorgänge von 10 s Dauer entzündet, die Brenneigenschaften nach jedem Zündvorgang werden beobachtet und danach die Probe bewertet. Zum Entzünden der Probe wird ein Bunsenbrenner mit einer 10 mm (3/8 inch) hoben blauen Flamme von Erdgas mit einem Wärmeinhalt von 3,73 × $10^4$ kJ/$m^3$ (1,000 BTU per cubic foot) benutzt.

Die UL 94 V 0-Klassifizierung umfaßt die nachstehend beschriebenen Eigenschaften von Materialien, die gemäß der UL 94 Vorschrift geprüft wurden. Die Polycarbonate in dieser Klasse enthalten keine

Proben, die länger als 10 s nach jeder Einwirkung der Testflamme brennen sie zeigen keine Gesamt-Flammzeit von mehr als 50 s bei der zweimaligen Flammeinwirkung auf jeden Probensatz ; sie enthalten keine Proben, die vollständig bis hinaus zu der am oberen Ende der Probe befestigten Halteklammer abbrennen ; sie weisen keine Proben auf, die die unterhalb der Probe angeordnete Watte durch brennende Tropfen oder Teilchen entzünden ; sie enthalten auch keine Proben, die länger als 30 s nach Entfernen der Testflamme glimmen.

Andere UL 94-Klassifizierungen bezeichnen Proben, die weniger flammwidrig und selbstverlöschend sind und die flammende Tropfen oder Teilchen abgeben. Diese Klassifizierungen werden mit UL 94 V-1 und V-2 bezeichnet. Die Polycarbonate innerhalb des Bereichs dieser Erfindung zeigen in charakteristischer Weise die für eine UL 94 V 0-Klassifizierung geforderten Eigenschaften.

## Beispiele

A. Herstellung eines Phthalimids der allgemeinen Formel (Ia)

A. 1N-(3,5-Bis-trifluormethyl-phenyl)-phthalimid

In einem Dreihalskolben, versehen mit Thermometer, Rührer und Wasserabscheider, werden 800 ml Eisessig vorgelegt und auf 55 °C erwärmt. Sodann wird eine Mischung aus 44,4 g Phthalsäureanhydrid (0,3 mol) und 68,7 g destilliertem 3,5-Bis-trifluormethylanilin (0,3 mol) zugegeben und kräftig gerührt. Beim Aufwärmen auf 108 °C wird eine klare Lösung erhalten. Diese wird für 2 1/2 Std. bei 117 °C unter Rückfluß gehalten und anschließend abgekühlt. Zu der Reaktionsmischung werden 100 ml Cyclohexan gegeben und die Mischung für 5 h bei 93 °C unter Rückfluß gehalten, wobei das Reaktionswasser im Wasserabscheider abgenommen werden kann.

Der Eisessig wird abdestilliert und das Endprodukt in Toluol aufgekocht, heiß filtriert und abgekühlt. Die ausgefallenen Kristalle werden mit Petrolether aufgeschlämmt, abgesaugt und getrocknet.

% N theor. : 3,91 ; %C theor. : 53,6 ; %H Theor. : 1,96
% N gef. : 3,85 ; %C gef. : 53,3 ; % H gef. : 1,84

B. Flammwidriges, verzweigtes Polycarbonat (Vergleich)

Ein verzweigtes Polycarbonat auf Basis Bisphenol A, 0,5 Mol-% 3,3-Bis-(4-hydroxy-3-methyl-phenyl)-2-oxo-2,3-dihydroindol, 3,0 Mol-% Phenol als Kettenabbrecher und Phosgen mit einer Lösungsviskosität von 1,31 (gemessen in $CH_2Cl_2$ bei 25 °C und in einer Konzentration von 0,5 g/100 ml) wurde mit 0,1 % n-Perfluorbutylsulfonsäure-K-Salz vermischt, extrudiert und gemäß UL 94 in einer Dicke von 3,2 mm und 1,6 mm auf seine Bandwidrigkeit untersucht.

Ergebnis : 3,2 mm V 0
1,6 mm V 2

C. Erfindungsgemäße flammwidriges, verzweigtes Polycarbonat

Ein verzweigtes Polycarbonat auf Basis Bisphenol A, 0,5 Mol-% 3,3-Bis-(4-hydroxy-3-methyl-phenyl)-2-oyo-2,3-dihydroindol, 3,0 Mol-% Phenol als Kettenabbrecher und Phosgen mit einer Lösungsviskosität von 1,31 (gemessen in $CH_2Cl_2$ bei 25 °C und in einer Konzentration von 0,5 g/100 ml) wurde mit 0,1 % n-Perfluorbutansulfonsäure-K-Salz und 0,5 % N-(3,5-Bis-trifluormethylphenyl)-phthalimid gemäß Beispiel A1 vermischt, extrudiert und gemäß UL 94 in einer Dicke von 3,2 mm, 1,6 mm und 0,8 mm auf seine Brandwidrigkeit untersucht.

Ergebnis : 3,2 mm V 0
1,6 mm V 0
0,8 mm V 1

## Patentansprüche

1. Phthalimide der Formel (I)

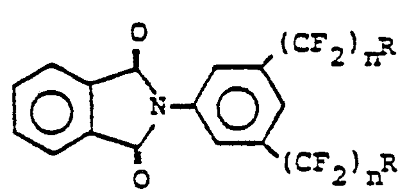

(I)

worin
n eine ganze Zahl von 1-12 und
R = H oder F sind, wobei für
n = 1 R nicht F ist.

2. Verfahren zur Herstellung von Phthalimiden der Formel I, dadurch gekennzeichnet, daß Phthalsäureanhydrid mit einem Amin der Formel II

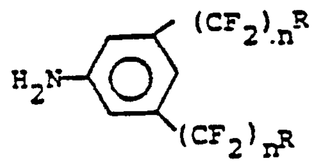

(II)

worin
n eine ganze Zahl von 1-12 und
R = H oder F sind, wobei für n = 1 R nicht F ist,
in äquimolaren Mengen bei etwa 117 °C und unter Mitverwendung von Eisessig als Lösungsmittel und Cyclohexan als Wasserschlepper umgesetzt wird.

3. Flammschutzmittelkombinationen bestehend aus
a) 0,1 bis 1 Gewichtsteil eines Phthalimids der Formel (Ia)

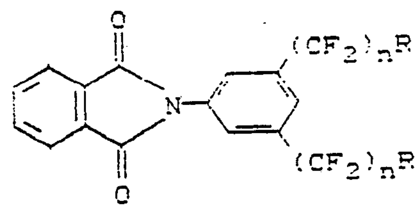

(Ia)

worin
n eine ganze Zahl von 1-12 und
R = H oder F
sind, und
b) 0,02 bis 2 Gewichtsteilen eines Alkalisalzes einer organischen oder anorganischen Säure.

4. Verwendung der Flammschutzmittelkombination des Anspruchs 3 zur Flammfestausrüstung von thermoplastischen, verzweigten, aromatischen Polycarbonaten aus halogenfreien, phenolischen Komponenten in Mengen von 0,1 bis 1 Gew.-%, bezogen auf Polycarbonat, an Phthalimid der Formel (Ia) des Anspruchs 3 und 0,02 bis 2 Gew.-%, bezogen auf Polycarbonat, an Alkalisalz einer organischen oder anorganischen Säure.

5. Verfahren zur Flammfestausrüstung von thermoplastischen, verzweigten, aromatischen Polycarbonaten aus halogenfreien, phenolischen Komponenten, dadurch gekennzeichnet, daß die Einarbeitung der Flammschutzmittelkombination des Anspruches 3 als Einzelkomponenten oder als Gemisch durch Mischen und anschließende Granulierung über einen Doppelwellenextruder bei einer Massetemperatur von 280-310 °C, vorzugsweise 290-300 °C erfolgt und bei einer Umdrehungszahl von 80-100 Umdrehungen/min ein Durchsatz von 5 kg/h erreicht wird.

6. Thermoplastische Formmassen auf Basis aromatischer, verzweigter, thermoplastischer Polycarbonate aus halogenfreien, phenolischen Komponenten mit einem Gehalt von 0,1 bis 1 Gew.-% Phthalimid der Formel (Ia) des Anspruchs 3 und von 0,02 bis 2 Gew.-% eines Alkalisalzes einer anorganischen oder organischen Säure, wobei sich die beiden Bereiche der Gewichtsprozente jeweils auf thermoplastisches, verzweigtes, aromatisches Polycarbonat ohne sonstige Zusätze beziehen.

7. Thermoplastische Formmassen gemäß Anspruch 6, dadurch gekennzeichnet, daß das thermoplastische Polycarbonat ein verzweigtes Polycarbonat auf Basis Bisphenol A mit einem Verzweigergehalt von 0,3 bis 1,0 Mol-%, bezogen auf Bisphenol A ist.

**Claims**

1. Phthalimides of the formula (I)

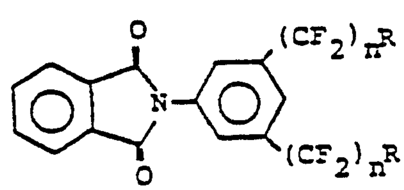

(I)

wherein

n is an integer from 1-12 and
R is H or F, but if
n = 1 R is not F.

2. Process for the preparation of phthalimides of the formula I, characterised in that phthalic anhydrides is reacted with an amine of the formula II

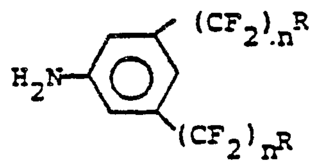

(II)

wherein

n is an integer from 1-12 and
R is H or F, but if
n = 1 R is not F,

in equimolar quantities at about 117 °C and with the concomitant use of glacial acetic acid as the solvent and cyclohexane as the water entrainer.

3. Flameproofing agent combinations consisting of

a) 0.1 to 1 part by weight of a phthalimide of the formula (Ia)

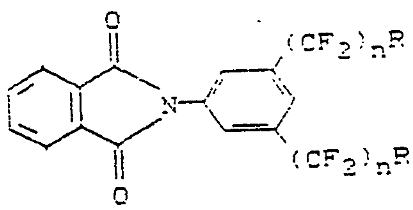

(Ia)

wherein

n is an integer from 1-12 and
R is H or F,

and

b) 0.02 to 2 parts by weight of an alkali metal salt of an organic or inorganic acid.

4. Use of the flameproofing agent combination of Claim 3 for flameproofing thermoplastic, branched, aromatic polycarbonates obtained from halogen-free, phenolic components in quantities of 0.1 to 1 % by weight, based on the polycarbonate, of a phthalimide of the formula (Ia) of Claim 3 and 0.02 to 2 % by weight, based on the polycarbonate, of an alkali metal salt of an organic or inorganic acid.

5. Process for flameproofing thermoplastic, branched, aromatic polycarbonates obtained from halogen-free, phenolic components, characterised in that the flameproofing agent combination of Claim 3 is incorporated in the form of the individual components or in the form of a mixture, by mixing and subsequent granulation via a twin-screw extruder at a stock temperature of 280-310 °C, preferably 290-300 °C and a throughtput of 5 kg/h is achieved at a speed of rotation of 80-100 revolutions/min.

6. Thermoplastic moulding compositions based on aromatic, branched, thermoplastic polycarbonates obtained from halogenfree phenolic components with a content of 0.1 to 1 % by weight of a phthalimide of the formula (Ia) of Claim 3 and 0.02 to 2 % by weight of an alkali metal salt of an inorganic or organic acid, the two ranges of the weight percentages relating in each case to the thermoplastic, branched, aromatic polycarbonate without any other additives.

7. Thermoplastic moulding compositions according to Claim 6, characterised in that the thermoplastic polycarbonate is a branched polycarbonate based on bisphenol A with a branching agent content of 0.3 to 1.0 mol %, based on bisphenol A.

**Revendications**

1. Phtalimides de formule (I)

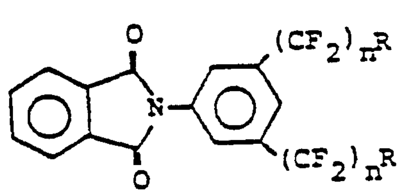

(I)

dans laquelle

n est un nombre entier de 1 à 12 et

R représente H ou F, sous réserve que lorsque n = 1, R ne représente pas F.

2. Procédé de production de phtalimides de formule I, caractérisé en ce qu'on fait réagir l'anhydride d'acide phtalique avec une amine de formule (II)

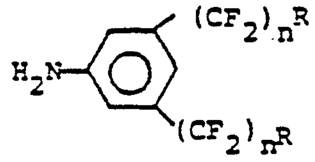

dans laquelle

n est un nombre entier de 1 à 12 et

R représente H ou F, sous réserve que lorsque n = 1, R ne représente pas F,

en quantités équimolaires à environ 117 °C et en utilisant simultanément de l'acide acétique cristallisable comme solvant et du cyclohexane comme substance entraînant l'eau.

3. Mélanges retardateurs de flamme, constitués

a) de 0,1 à 1 partie en poids d'un phtalimide de formule (Ia)

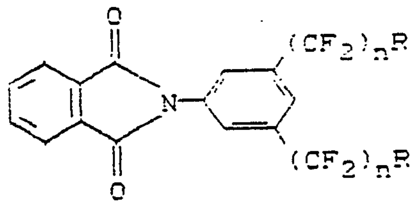

(Ia)

dans laquelle

n est un nombre entier de 1 à 12, et

R représente H ou F,

et

b) de 0,02 à 2 parties en poids d'un sel alcalin d'un acide organique ou inorganique.

4. Utilisation du mélange retardateur de flamme suivant la revendication 3, pour l'ignifugeage de polycarbonates aromatiques thermoplastiques ramifiés dérivés de composants phénoliques non halogénés, dans des quantités de 0,1 à 1% en poids, par rapport au polycarbonate, de phtalimide de formule (Ia) suivant la revendication 3, et de 0,02 à 2 % en poids, par rapport au polycarbonate, d'un sel alcalin d'un acide organique ou inorganique.

5. Procédé d'ignifugeage de polycarbonates aromatiques thermoplastiques ramifiés, dérivés de composants phénoliques non halogénés, caractérisé en ce que l'incorporation du mélange retardateur de flamme suivant la revendication 3 comme composant individuel ou coma mélange est effectuée par mélange, puis granulation sur une extrudeuse à deux arbres à une température du mélange de 280 à 310 °C, de préférence de 290 à 300 °C, et un débit de 5 kg/h est atteint pour une vitesse de rotation de 80 à 100 tours par minute.

6. Mélanges thermoplastiques à mouler à base de polycarbonates aromatiques thermoplastiques ramifiés, dérivés de composants phénoliques non halogénés, ayant une teneur de 0,1 à 1 % en poids en phtalimide de formule (Ia) suivant la revendication 3 et une teneur de 0,02 à 2 % en poids d'un sel alcalin d'un acide organique ou inorganique, les deux intervalles de pourcentages en poids se rapportant chacun au polycarbonate aromatique thermoplastique ramifié sans autres additifs.

7. Mélanges à mouler thermoplastiques suivant la revendication 6, caractérisés en ce que le polycarbonate thermoplastique est un polycarbonate ramifié à base de Bisphénol A ayant une teneur en groupes ramifiants de 0,3 à 1,0 mole % par rapport au Bisphénol A.